(19) Europäisches Patentamt
European Patent Office
Office européen des brevets

(11) EP 3 582 767 B1

(12) **FASCICULE DE BREVET EUROPEEN**

(45) Date de publication et mention de la délivrance du brevet:
**07.04.2021 Bulletin 2021/14**

(21) Numéro de dépôt: **18708058.5**

(22) Date de dépôt: **13.02.2018**

(51) Int Cl.:
*A61K 31/216* (2006.01) *A61K 31/7048* (2006.01)
*A61P 3/04* (2006.01) *A61P 3/06* (2006.01)
*A61P 3/10* (2006.01)

(86) Numéro de dépôt international:
**PCT/EP2018/053520**

(87) Numéro de publication internationale:
**WO 2018/149812 (23.08.2018 Gazette 2018/34)**

(54) **PRINCIPE ACTIF PHARMACEUTIQUE ET UTILISATION EN PARTICULIER DANS LA PREVENTION ET LE TRAITEMENT DES DEREGLEMENTS METABOLIQUES CHEZ L'HOMME ET L'ANIMAL**

PHARMAZEUTISCHE WIRKSTOFFSUBSTANZ UND VERWENDUNG DAVON, INSBESONDERE ZUR PRÄVENTION UND BEHANDLUNG VON STOFFWECHSELSTÖRUNGEN BEI MENSCHEN UND TIEREN

PHARMACEUTICAL ACTIVE INGREDIENT AND USE THEREOF, IN PARTICULAR FOR THE PREVENTION AND TREATMENT OF METABOLIC DISORDERS IN HUMANS AND ANIMALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **16.02.2017 FR 1770144**

(43) Date de publication de la demande:
**25.12.2019 Bulletin 2019/52**

(73) Titulaires:
- **Valbiotis**
  **17180 Perigny (FR)**
- **Université Clermont Auvergne**
  **63000 Clermont-Ferrand (FR)**
- **La Rochelle Université**
  **17071 La Rochelle Cedex 9 (FR)**
- **CNRS**
  **75794 Paris Cedex 16 (FR)**

(72) Inventeurs:
- **PELTIER, Sébastien**
  **17450 Fouras (FR)**
- **CHAVANELLE, Vivien**
  **63000 Clermont-Ferrand (FR)**
- **LE JOUBIOUX, Florian**
  **17440 Aytre (FR)**
- **SIRVENT, Pascal**
  **63122 Ceyrat (FR)**
- **MAUGARD, Thierry**
  **17220 La Jarne (FR)**

(74) Mandataire: **Aquinov**
**Allée de la Forestière**
**33750 Beychac et Caillau (FR)**

(56) Documents cités:
**WO-A1-2009/118380**

- **JIN SHASHA ET AL: "Chlorogenic Acid Improves Late Diabetes through Adiponectin Receptor Signaling Pathways in db/db Mice", PLOS ONE, vol. 10, no. 4, avril 2015 (2015-04), XP002773403,**
- **GARAMBONE E ET AL: "Possíveis benefícios do ácido clorogênico à saúde - Possible health benefits of chlorogenic acid.", ALIMENTOS E NUTRIÇÃO ARARAQUARA, vol. 18, no. 2, juin 2007 (2007-06), pages 229-235, XP002773407, ISSN: 0103-4235**
- **ZHANG J ET AL: "Efficient method for the screening and identification of anti-diabetic components in the leaves of Olea europaea L.", NEW JOURNAL OF CHEMISTRY AUGUST 201 ROYAL SOCIETY OF CHEMISTRY GBR, vol. 38, no. 8, août 2014 (2014-08), pages 3796-3802, XP002773404, ISSN: 1144-0546**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **ANDREADOU IOANNA ET AL: "The olive constituent oleuropein exhibits anti-ischemic, antioxidative, and hypolipidemic effects in anesthetized rabbits", THE JOURNAL OF NUTRITION, AMERICAN SOCIETY FOR NUTRITION, US, vol. 136, no. 8, 1 août 2006 (2006-08-01), pages 2213-2219, XP002575927, ISSN: 0022-3166**

## Description

La présente invention a pour objet un principe actif pharmaceutique constitué par l'association de deux molécules, et son utilisation comme médicament, en particulier dans la prévention et/ou le traitement des dérèglements pathologiques du métabolisme glucidique et/ou lipidique.

Les troubles et maladies métaboliques chroniques sont de plus en plus fréquentes. C'est le cas notamment du diabète de type 2, qui est devenu un enjeu médical majeur dans le monde entier (Boyle JP et al., Popul Health Metr 2010;8:29). En effet, selon la Fédération Internationale du Diabète (FID), le nombre de diabétiques de type 2 était de 415 millions en 2015 et atteindrait 642 millions d'ici 2040, soit une hausse de 55% (Atlas du diabète de la FID, 7ème édition). En 2015, le diabète a causé 5 millions de décès, soit un décès toutes les six secondes, et les dépenses de santé mondiale dues à cette pathologie étaient de 673 milliards de dollars.

Le diabète de type 2 est caractérisé par une concentration en sucre dans le sang anormalement élevée et une intolérance aux glucides. La principale cause de cet état hyperglycémique chronique est une résistance à l'insuline ainsi qu'une sécrétion inadéquate en réponse à un état métabolique donné (Beigi FI, N Eng J Med 2012;366:1319-27). Pour lutter contre cette pathologie, il convient principalement de réduire les niveaux de glycémie et de diminuer l'hémoglobine glyquée ($HbA_1c$) à un taux inférieur ou égal à 7.0% pour les adultes (hors femmes enceintes; ADA, Diabetes Care 2015;38(1):S33-S40). La grande majorité des patients diagnostiqués sont sous traitement pharmacologique pour le reste de leur vie (Qaseem A et al., Ann Intern Med 2012;156:218-31). Pour autant, les thérapeutiques actuelles ne sont pas effectives pour corriger les causes du diabète de type 2 (Tao H et al., Nat Med 2014;20(11):1263-69), en particulier l'insulino-résistance et la perte de la capacité du pancréas à sécréter de l'insuline au regard de l'augmentation de la glycémie. Ainsi, la plupart des patients deviennent réfractaires aux traitements actuels (Qaseem A et al., Ann Intern Med 2012;156:218-31; Nathan D et al., Diabetes Care 2009;32:193-203). Le développement de nouveaux médicaments, en particulier ceux capables de maintenir davantage une sécrétion d'insuline en réponse à une augmentation de la glycémie constitue une priorité pour lutter contre le développement du diabète de type 2 et de ses complications. Par ces effets, de tels médicaments permettraient de retarder, voire de ne pas instaurer, une insulino-thérapie. Chez les patients diabétiques, on observe également une prévalence importante de la NAFLD qui varie de 50 à 70% (Anstee Q et al. Nat Rev Gastroenterol Hepatol 2013;10:330-44 ; Targher G et al. Diabetes Care 2007;30:1212-8 ; Williamson R et al. Diabetes Care 2011;34:1139-44). La NAFLD ou stéatose hépatique non-alcoolique (équivalent du terme anglais « *non alcoholic fatty liver disease* ») englobe un spectre allant de la stéatose hépatique simple jusqu'à la stéatohépatite non alcoolique (équivalent du terme anglais « *nonalcoholic steatohepatitis* », NASH) et la cirrhose (Angulo P N Eng J Med 2002;346:1221-31 ; Neuschwander-Tetri B et al. Hepatology 2003;37:1202-19 ; Adams L et al. Gastroenterology 2005;129:113-21 ; Kotronen A et al. J Clin Endocrinol Metab 2007;92:3490-7). La NAFLD est caractérisée par une accumulation intra-hépatique excessive de graisses (stéatose), qui peut être isolée ou associée à une inflammation hépatique non-spécifique. La NASH est la forme progressive de NAFLD et elle est définie par l'association d'une stéatose dans plus de 5% des hépatocytes à une inflammation lobulaire et à des lésions de souffrance hépatocytaire (ballonnisation) (Adams L et al. Cmaj 2005;172:899-905 ; Kleiner D et al. Hepatology 2005;41:1313-21 ; Brunt E Nat Rev Gastroenterol Hepatol 2010;7:195-203). La stéatose simple touchant entre 1 et 5% des hépatocytes est considérée comme physiologique et elle est une évolution bénigne, alors que la NASH est définie par une atteinte hépatocytaire, l'inflammation et/ou une fibrose qui peut conduire à la cirrhose, l'insuffisance hépatique et le carcinome hépatocellulaire (Vanni E et al. Dig Liver Dis 2010;42:320-30).

Par ailleurs, les patients diabétiques de type 2 ont un risque élevé de morbi-mortalité cardiovasculaire. Il est donc également nécessaire de prendre en charge les facteurs de risque cardiovasculaire traditionnels comme notamment le contrôle des lipides circulants et du poids. Cette nécessité induit actuellement la prise de plusieurs médicaments de différentes classes thérapeutiques simultanément. Or, la combinaison de drogues peut parfois engendrer des réactions secondaires graves comme par exemple, l'administration simultanée de fibrates et de statines qui augmente le risque de myopathie (Denke M J Manag Care Pharm 2003;9:17-9).

Il existe donc un besoin urgent de médicaments dont le mécanisme d'action « multi-cibles » présente des avantages en termes de compliance, de tolérance et d'efficacité. De tels produits permettraient de diminuer le risque global de maladies cardio-métaboliques et de prévenir et traiter chaque dysfonctionnement et/ou ses conséquences pris indépendamment.

Un objectif de la présente invention est de proposer un principe actif répondant à ces besoins médicaux non satisfaits, qui soit en particulier capable de maintenir une sécrétion d'insuline adéquate en réponse à l'augmentation de la glycémie lors du développement du diabète de type 2.

Il est important de préciser que dans la majorité des cas, chez un patient adulte, la NAFLD et la NASH sont associées à une insulino-résistance et ses complications phénotypiques, essentiellement les affections faisant partie du syndrome métabolique : le diabète de type 2, l'obésité, l'hypertension artérielle, l'hypercholestérolémie et l'hypertriglycéridémie (Marchesini G et al. Diabetes 2001;50:1844-50; Ratziu V et al. J Hepatol 2010;53:372-84; Neuschwander-Tetri BA et al. Hepatology 2003;37:1202-19).

Un autre objectif de la présente invention est donc de proposer un principe actif capable :

- de diminuer l'insulino-résistance ;
- de diminuer le stress oxydant et l'inflammation ;
- de limiter le développement des adipocytes ;
- de diminuer le taux de triglycérides circulants.

Pour répondre à ses objectifs, l'invention vise l'utilisation d'un principe actif pharmaceutique constitué exclusivement par l'association :

- de l'acide (1S,3R,4R,5R)-3-{[(2E)-3-(3,4-dihydroxyphényl)-2-propenoyl]oxy}-1,4,5-trihydroxycyclohexanecarboxylique, et
- du (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle).

Des extraits végétaux contenant ces molécules ont déjà été décrits dans la littérature pour des applications dans des produits de nutrition et des compositions pharmaceutiques mais de façon surprenante, les résultats obtenus avec le principe actif selon l'invention sont différents de ceux que l'on pourrait obtenir avec des extraits végétaux contenant ces deux molécules. Le principe actif est constitué uniquement par ces deux molécules qui agissent en synergie en particulier pour diminuer la glycémie à jeun, et limiter la perte de la capacité du pancréas à sécréter de l'insuline au regard de l'augmentation de la glycémie. Avantageusement, le principe actif selon l'invention permet également d'envisager une instauration plus tardive de l'insulino-thérapie se traduisant pour les patients par une amélioration considérable de la qualité de vie (injections d'insuline retardées, voire évitées).

Avantageusement, un tel principe actif permet donc de limiter voire d'éviter l'insulino-résistance, mécanisme important du développement du diabète de type 2.

De plus, le principe actif selon l'invention est capable d'agir sur le stress oxydant, l'inflammation, et sur le métabolisme lipidique en diminuant notamment le développement des adipocytes et les triglycérides sériques.

Par conséquent l'invention a pour objet un principe actif pharmaceutique constitué exclusivement par l'association de l'acide (1S,3R,4R,5R)-3-{[(2E)-3-(3,4-dihydroxyphényl)-2-propenoyl]oxy}-1,4,5-trihydroxycyclohexanecarboxylique et de (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle, pour une utilisation comme médicament ou produit vétérinaire, notamment pour prévenir et/ou traiter des dérèglements pathologiques du métabolisme glucidique et/ou lipidique chez l'Homme ou l'animal.

L'invention vise également les compositions pharmaceutiques comprenant un principe actif constitué exclusivement par l'association de l'acide (1S,3R,4R,5R)-3-{[(2E)-3-(3,4-dihydroxyphényl)-2-propenoyl]oxy}-1,4,5-trihydroxycyclohexanecarboxylique et du (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle pour cette même utilisation.

L'invention est à présent décrite en détail en regard des figures annexées qui représentent :

- Figure 1 : une illustration du développement du diabète de type 2 chez la souris ;
- Figure 2 : les résultats de l'essai présenté au point II, relatifs à la prise alimentaire après 5 semaines de traitement (valeurs moyennes), ces résultats correspondant aux résultats du Tableau 1 ;
- Figure 3 : les résultats de l'essai présenté au point II, relatifs à la glycémie à jeun (valeurs moyennes) après 6 semaines de traitement, ces résultats correspondant aux résultats du Tableau 2 ;
- Figure 4 : les résultats de l'essai présenté au point II, relatifs au facteur de synergie après 6 semaines de traitement ;
- Figure 5 : les résultats de l'essai présenté au point II, relatifs à l'insulinémie à jeun après 6 semaines de traitement (valeurs médianes), ces résultats correspondant aux résultats du Tableau 3 ;
- Figure 6 : les résultats de l'essai présenté au point II, relatifs aux triglycérides sériques à jeun après 6 semaines de traitement (valeurs moyennes), ces résultats correspondant aux résultats du Tableau 4.

L'invention a pour objet un principe actif pharmaceutique constitué exclusivement par l'association de l'acide (1S,3R,4R,5R)-3-{[(2E)-3-(3,4-dihydroxyphényl)-2-propenoyl]oxy}-1,4,5-trihydroxycyclohexanecarboxylique et de (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle pour une utilisation comme médicament ou produit vétérinaire.

L'acide (1S,3R,4R,5R)-3-{[(2E)-3-(3,4-dihydroxyphényl)-2-propenoyl]oxy}-1,4,5-trihydroxycyclohexanecarboxylique correspond à la formule suivante :

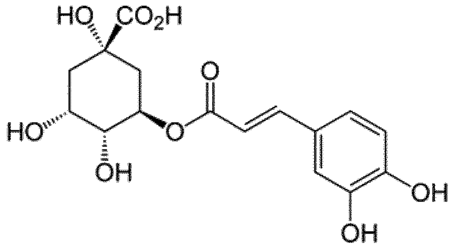

Il peut être désigné par le terme « V63X35 » dans les exemples et essais présentés dans la présente demande.

Le (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle) correspond à la formule suivante :

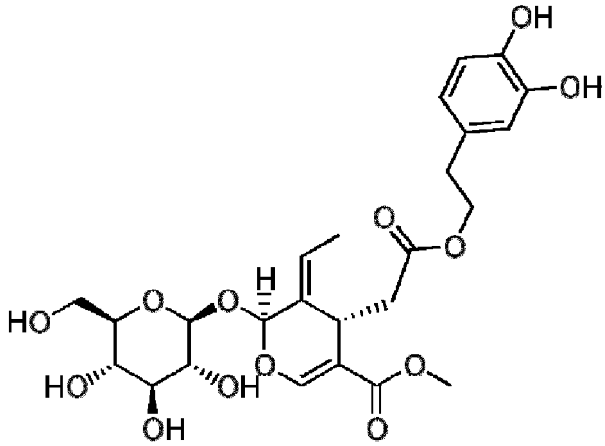

Il peut être désigné par le terme « V63X54 » dans les exemples et essais présentés dans la présente demande.

Ces deux molécules constituant le principe actif selon l'invention, sont des molécules naturelles ou de synthèse chimique et/ou biotechnologique. Par molécules naturelles, on entend des molécules extraites d'une matière première végétale avec une pureté comprise entre 80% et 100%.

Le principe actif est exclusivement constitué par ces deux molécules et n'en comporte pas d'autres à l'exception des éventuelles impuretés dans le cas de molécules extraites de matière première végétale.

Préférentiellement, le ratio acide (1S,3R,4R,5R)-3-{[(2E)-3-(3,4-dihydroxyphényl)-2-propenoyl]oxy}-1,4,5-trihydroxycyclohexanecarboxylique sur (2S,3E,4S)-4-{2-[2-(3,4-Dihydroxyphényl)éthoxy]-2-oxoéthyl}-3-éthylidène-2-(P-D-glucopyranosyloxy)-3,4-dihydro-2H-pyrane-5-carboxylate de méthyle en poids est compris entre 1/40 et 1/1, encore plus préférentiellement entre 1/1 et 1/10. En effet, ces ratios permettent d'obtenir une efficacité optimale pour la prévention et/ou le traitement des dérèglements du métabolisme glucidique et/ou lipidique, en particulier dans la lutte contre le diabète de type 2.

Le principe actif selon l'invention peut se présenter sous forme sèche de manière à être pris par voie orale, la forme sèche permettant la réalisation de galéniques solides comme le comprimé ou la gélule. Le principe actif selon l'invention peut également se présenter sous forme liquide pour une utilisation par voie orale, entérale, parentérale ou sous-cutanée.

Le principe actif selon l'invention peut être obtenu par tout procédé adapté. Il peut notamment être obtenu par simple mélange des deux molécules dans les proportions désirées. Préférentiellement, les molécules constituant le principe actif selon l'invention est obtenu par un procédé de synthèse chimique et/ou biotechnologique des deux molécules. La synthèse de l'acide (1S,3R,4R,5R)-3-{[(2E)-3-(3,4-dihydroxyphényl)-2-propenoyl]oxy}-1,4,5-trihydroxycyclohexanecarboxylique peut être réalisée par le couplage chimique et/ou enzymatique du (2E)-3-(3,4-dihydroxyphényl)acrylate avec l'acide (1S,3R,4S,5R)-1,3,4,5-tétrahydroxycyclohexanecarboxylique. La réaction d'estérification peut être réalisée de préférence dans un milieu dit non conventionnel par un catalyseur enzymatique type lipase. La synthèse du (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle, peut être réalisée par le couplage chimique et/ou enzymatique du 4-(2-Hydroxyéthyl)-1,2-benzènediol avec l'acide [(2S,3S,4S)-3-formyl-5-(méthoxycarbonyl)-2-méthyl-3,4-dihydro-2H-pyran-4-yl]acétique, préalablement glucosylé. La réaction d'esterification et de glucosylation peuvent être réalisée de préférence dans un milieu dit non conventionnel par des catalyseurs enzymatiques, une lipase et une β-D-glucosidase respectivement.

Préférentiellement, le principe actif selon l'invention est utilisé dans une composition pharmaceutique.

L'invention a donc également pour objet une composition pharmaceutique comprenant au moins un principe actif tel que décrit précédemment, pour une utilisation comme médicament ou produit vétérinaire. Une telle composition peut se présenter sous forme solide ou liquide, en fonction du mode et de la forme d'administration choisie pour le principe actif.

Le principe actif selon l'invention est préférentiellement présent entre 0,05% (par exemple une forme comprimée avec des excipients) et 100% en poids de matière sèche de la composition finale (hors support, par exemple un principe actif à hauteur de 100% de la composition au sein d'une gélule).

Le poids en matière humide du principe actif en pourcentage de la composition finale dépend de la nature des excipients utilisés.

La composition, en plus du principe actif selon l'invention, quand elle se présente sous forme sèche ou sous forme sèche à reconstituer dans de l'eau, peut éventuellement comprendre au moins un excipient adapté à cette forme, choisi parmi par exemple, sans que cette liste soit exhaustive : acide stéarique, amidon de maïs, cellulose microcristalline, citral, croscarmellose sel de Na, crospovidone, fer jaune oxyde, gélatine, géraniol, gomme xanthane, hyprolose, hypromellose, indigotine, macrogol 400, macrogol 8000, magnésium stéarate, maltodextrine, opadry cler, povidone K 30, propylèneglycol, sillice colloïdale anhydre, sodium carboxyméthylamidon, sodium benzoate, titane dioxyde.

La composition, en plus du principe actif selon l'invention, quand elle se présente sous forme liquide, peut éventuellement comprendre au moins un excipient adapté à cette forme, choisi parmi par exemple, sans que cette liste soit exhaustive : alcool benzylique, carmellose sodique, cétylpyridinium chlorure, eau ppi, glycérol, métacrésol, phénol, phosphate monosodique dihydraté, phosphate disodique dihydrate, phosphate disodique dodécahhydraté, propylèneglycol, polysorbate 80, protéines d'*Escherichia coli,* sodium chlorure, sodium hydroxyde, zinc chlorure.

Le principe actif pharmaceutique et la composition selon l'invention peuvent en particulier être utilisés dans la prévention et/ou le traitement des dérèglements pathologiques du métabolisme glucidique et/ou lipidique chez l'Homme ou l'animal.

Par dérèglement du métabolisme pathologique au sens de l'invention, on entend tous dérèglements favorisant et/ou aggravant et/ou à l'origine de pathologies métaboliques. Les affections métaboliques forment un environnement pathologique regroupant des désordres multifactoriels plus ou moins liés par une origine, des cibles métaboliques ou des mécanismes communs.

Par dérèglement du métabolisme glucidique au sens de l'invention on entend modification du métabolisme cellulaire des glucides pouvant entraîner et/ou favoriser et/ou aggraver des pathologies. Par exemple, le diabète de type 2 est associé à un dérèglement du métabolisme glucidique avec notamment une élévation de la production hépatique de glucose.

Par dérèglement du métabolisme lipidique au sens de l'invention on entend modification du métabolisme cellulaire des lipides pouvant entraîner et/ou favoriser et/ou aggraver des pathologies. Par exemple, la NAFLD est associée à un dérèglement du métabolisme lipidique avec notamment une accumulation intra-hépatique excessive de graisses.

Le principe actif et la composition selon l'invention sont notamment efficaces dans la prévention et/ou le traitement d'au moins une maladie choisie parmi :

- le diabète de type 2, en maintenant une sécrétion d'insuline adéquate en réponse à l'augmentation de la glycémie lors du développement de la maladie ;
- les maladies du foie gras non alcoolique, en particulier la NAFLD et la NASH, en agissant sur l'insulino-résistance, le stress oxydant et l'inflammation ;
- la dyslipidémie, en particulier en diminuant les triglycérides sériques ;
- l'obésité en diminuant le développement des adipocytes ;
- le syndrome métabolique,
- les pathologies cardiovasculaires, en particulier les pathologies cardiovasculaires issues des complications du diabète de type 2 et/ou de la NAFLD ou de la NASH et/ou de la dyslipidémie et/ou de l'obésité et/ou d'un syndrome métabolique, en particulier celles choisies parmi les cardiopathies coronariennes, les maladies cérébro-vasculaires, les artériopathies périphériques et les thromboses veineuses profondes.

Pour ces effets, les deux molécules constituant le principe actif selon l'invention agissent en synergie.

Lorsqu'il est administré à l'Homme, par voie orale, notamment chez des patients souffrant de diabète de type 2, le principe actif selon l'invention est préférentiellement administré à raison de 5 à 5000 mg/jour, encore plus préférentiellement de 100 à 3000 mg/jour. La dose par voie entérale, parentérale ou intrapéritonéale est préférentiellement inférieure d'au moins un facteur 5.

Lorsqu'il est administré au chien ou au chat, par voie orale, en particulier chez le chien ou le chat souffrant de diabète de type 2, le principe actif selon l'invention est préférentiellement administré à raison de 1 à 5000 mg/jour, encore plus préférentiellement de 10 à 2000 mg/jour. La dose par voie entérale, parentérale ou intrapéritonéale est préférentiellement inférieure d'au moins un facteur 5.

Avantageusement, le principe actif et la composition selon l'invention permettent de diminuer la glycémie, de maintenir une sécrétion d'insuline adéquate en réponse à l'augmentation de la glycémie lors du développement du diabète de type 2, d'améliorer la sensibilité à l'insuline, de diminuer l'inflammation et le stress oxydant, de diminuer les triglycérides sériques, et de réguler de manière bénéfique le développement des adipocytes. En particulier, les effets du principe actif selon l'invention sont supérieurs à ceux de la metformine, le médicament de référence dans le traitement du diabète de type 2, sur le métabolisme de l'insuline (Figure 5). Par ailleurs, la prise orale possible du principe actif et de la composition selon l'invention constitue un avantage certain par rapport à de nombreuses molécules antidiabétiques uniquement administrables par injection (exemples : dulaglutide, liraglutide, combo insuline glargine/lixisénatide).

L'invention est à présent illustrée par des exemples de principes actifs et de compositions, ainsi que par des résultats d'essais démontrant l'efficacité de l'invention, ces exemples et essais n'étant pas limitatifs.

I. EXEMPLES

Exemple 1 : exemple de principe actif selon l'invention

Le principe actif selon l'invention de l'exemple 1 est composé de la combinaison de l'acide (1S,3R,4R,5R)-3-{[(2E)-3-(3,4-dihydroxyphényl)-2-propenoyl]oxy}-1,4,5-trihydroxycyclohexanecarboxylique et du (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle selon un rapport de 1 / 4.6 sous forme sèche.

La synthèse de l'acide (1S,3R,4R,5R)-3-{[(2E)-3-(3,4-dihydroxyphényl)-2-propenoyl]oxy}-1,4,5-trihydroxycyclohexanecarboxylique a été réalisée par le couplage du (2E)-3-(3,4-dihydroxyphényl)acrylate avec l'acide (1S,3R,4S,5R)-1,3,4,5-tétrahydroxycyclohexanecarboxylique. Cette réaction d'estérification est préférentiellement réalisée par un procédé de couplage chimique et/ou biotechnologique des deux molécules. Préférentiellement, le couplage a été réalisé dans un milieu dit non conventionnel par un catalyseur enzymatique type lipase. Il s'agit préférentiellement de la lipase B de *Candida antarctica,* à une concentration de 20 g/L, pour catalyser en 12h le couplage de 150 mM de (2E)-3-(3,4-dihydroxyphényl)acrylate avec 150 mM de l'acide (1S,3R,4S,5R)-1,3,4,5-tétrahydroxycyclohexanecarboxylique dans un mélange 2-méthylbutan-2-ol/n-hexane 40/60 (v/v) à 55°C.

La synthèse du (2S,3E,4S)-4-{2-[2-(3,4-Dihydroxyphényl)éthoxy]-2-oxoéthyl}-3-éthylidène-2-(P-D-glucopyranosyloxy)-3,4-dihydro-2H-pyrane-5-carboxylate de méthyle, a été réalisée par le couplage chimique et/ou enzymatique du 4-(2-Hydroxyéthyl)-1,2-benzènediol avec l'acide [(2S,3S,4S)-3-formyl-5-(méthoxycarbonyl)-2-méthyl-3,4-dihydro-2H-pyran-4-yl]acétique, préalablement glucosylé. La réaction d'esterification et de glucosylation a été réalisée dans un milieu dit non conventionnel par des catalyseurs enzymatiques, une lipase et une β-D-glucosidase respectivement.

Préférentiellement, la lipase B de *Candida antarctica* est utilisée, à une concentration de 20 g/L, pour catalyser en 24h le couplage de 150 mM de 4-(2-Hydroxyéthyl)-1,2-benzènediol avec 75 mM de l'acide [(2S,3S,4S)-3-formyl-5-(méthoxycarbonyl)-2-méthyl-3,4-dihydro-2H-pyran-4-yl]acétique, préalablement glucosylé, dans un mélange 2-méthylbutan-2-ol/n-hexane 90/10 (v/v) à 55°C.

Préférentiellement, la glucosidase de *Sclerotinia sclerotiorum* est utilisée, à une concentration de 30 U/mL, pour catalyser en 10h la glucosylation de 50 mM de [(2S,3S,4S)-3-formyl-5-(méthoxycarbonyl)-2-méthyl-3,4-dihydro-2H-pyran-4-yl]acétique, avec 100 mM de pNP-β-D-glucopyranoside au sein d'un mélange DMSO/acétone 30/70 (v/v) à 40°C.

Exemple 2 : exemple de composition selon l'invention

La composition selon l'invention de l'exemple 2 comprend par dose journalière 100 mg de l'acide (IS,3R,4R,5R)-3-{[(2E)-3-(3,4-dihydroxyphényl)-2-propenoyl]oxy}-I,4,5-trihydroxycyclohexanecarboxylique et 460 mg de (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle sous forme sèche.

La composition selon l'invention se présente sous forme de comprimés blanc sécables de 1200 mg. Elle comprend les excipients suivants : hypromellose, magnésium stéarate, povidone K 30. Les excipients représentent donc 640 mg. Le principe actif selon l'invention représente 53,3% du poids total du comprimé, les excipients 46,7%. Le comprimé est réalisé selon les méthodes connues par l'homme du métier.

Exemple 3 : exemple de composition selon l'invention

La composition selon l'invention comprend par dose journalière 1 mg de l'acide (1S,3R,4R,5R)-3-{[(2E)-3-(3,4-dihydroxyphényl)-2-propenoyl]oxy}-1,4,5-trihydroxycyclohexanecarboxylique et 6 mg de (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle sous forme liquide. La composition selon l'invention se présente sous forme injectable avec comme excipients : alcool benzylique, camellose sodique, cétylpyridinium chlorure, eau ppi, polysorbate 80, sodium chlorure. La solution injectable est réalisée selon les méthodes connues par l'homme du métier.

II. EVALUATION IN VIVO DE l'EFFICACITE DE LA COMPOSITION

Des expérimentations *in vivo* sur des souris ont été réalisées pour démontrer les effets du principe actif selon l'invention, en particulier sur la glycémie à jeun et la capacité de sécrétion d'insuline en réponse à une augmentation chronique de la glycémie à jeun. La composition a également été comparée à la metformine, une des principales molécules pharmaceutiques prescrite pour le traitement du diabète de type 2.

Les expérimentations ont été réalisées sur des souris db/db, modèle mimant les caractéristiques du diabète de type 2 chez l'Homme (Roesler WJ et al., Mol Cell Biochem 1990;92(2):99-106). Ces souris sont insulino-résistantes, hypertriglycéridémiques, et intolérantes au glucose. Elles développent très rapidement un pré-diabète, un diabète de type 2 puis une NASH.

La Figure 1 illustre l'évolution de l'insulinémie et de la glycémie (glucose) au cours du temps chez ce modèle (Joost HG et al., Animal Models in Diabetes Research, Humana Press). Comme pour l'Homme, l'élévation de la glycémie s'accompagne dans un premier temps d'une augmentation de l'insulinémie (associée à une insulino-résistance), preuve d'un pancréas encore en capacité de sécréter de l'insuline, puis d'une diminution, traduisant une dégénérescence fonctionnelle du pancréas, et donc d'une progression du diabète de type 2 avec nécessité d'une insulino-thérapie (Kobayashi K et al., Metabolism: clinical and experimental 2000;49:22-31; Tao H et al. Nat Med 2014;20(11):1263-1269).

Le temps expérimental pour l'essai était de 6 semaines avec un « Run-in » d'1 semaine suivie de 6 semaines de traitement. Les souris mâles étaient âgées de 6 semaines en début de traitement. L'acide (1S,3R,4R,5R)-3-{[(2E)-3-(3,4-dihydroxyphényl)-2-propenoyl]oxy}-1,4,5-trihydroxycyclohexanecarboxylique seul, le (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle seul, et le mélange de ces deux molécules dans un ratio de 1 / 4,65 ont été testés et comparés à la metformine. Ces compositions étaient directement intégrées dans l'alimentation des rongeurs, permettant ainsi de s'assurer d'une utilisation à grande échelle, les injections, intraveineuses par exemple, pouvant être limitées à un faible nombre étant donné leur mode d'administration.

Après randomisation en fonction du poids et de la glycémie à jeun, les animaux étaient répartis dans les groupes suivants :

- Contrôle (n = 15) : CONTRÔLE ;
- Metformine (n = 12, 0.2% de l'alimentation) : MET ;
- l'acide (1S,3R,4R,5R)-3-{[(2E)-3-(3,4-dihydroxyphényl)-2-propenoyl]oxy}-1,4,5-trihydroxycyclohexanecarboxylique (n = 12, 0.026% de l'alimentation) : V63X35 ;
- le (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle (n = 11, 0.12% de l'alimentation) : V63X54 ;
- l'acide (1S,3R,4R,5R)-3-{[(2E)-3-(3,4-dihydroxyphényl)-2-propenoyl]oxy}-1,4,5-trihydroxycyclohexanecarboxylique + le (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle (n = 11, respectivement 0.026% et 0.12% de l'alimentation) : V63000.

Les souris étaient nourries *ad libitum* et avaient accès librement à l'eau. Elles étaient placées par ailleurs en cages individuelles. La prise alimentaire était mesurée chaque semaine sauf en fin de traitement (semaine 6) en raison des différentes évaluations réalisées.

Les évaluations expérimentales réalisées à jeun (6 h) après 6 semaines de traitement ont notamment porté sur :

- La mesure du poids ;
- La mesure de la glycémie ;
- La mesure de l'insulinémie ;
- La mesure des triglycérides sériques.

Les résultats sur la mesure de prise alimentaire sont présentés dans le Tableau 1 et sur la Figure 2.

***Tableau 1. Prise alimentaire après 6 semaines de traitement***

| CONTRÔLE | MET | V63X35 | V63X54 | V63000 |
|---|---|---|---|---|

(suite)

| CONTRÔLE | MET | V63X35 | V63X54 | V63000 |
|---|---|---|---|---|
| 7,7 ± 0,5 | 7,9 ± 0,5 | 7,6 ± 0,6 | 7,5 ± 0,5 | 7,7 ± 0,3 |
| Résultats exprimés en g/jour ; valeurs moyennes ± SEM (erreur type de la moyenne). | | | | |

On constate qu'aucune composition n'a induit de changement dans la prise alimentaire. Les résultats obtenus sont donc indépendants de la prise alimentaire. Selon la méthode de Reagan-Show S et al. (FASEB J 2008;22(3):659-61), la dose humaine équivalente de la combinaison de molécules ingérée par les rongeurs était de 19.4 mg/kg de poids corporel et par jour.

Les résultats de la mesure de la glycémie sont présentés dans le Tableau 2 et sur la Figure 3.

***Tableau 2. Glycémie à jeun après 6 semaines de traitement***

| CONTRÔLE | MET | V63X35 | V63X54 | V63000 |
|---|---|---|---|---|
| 481 ± 31 | 454 ± 34 | 454 ± 34 | 432 ± 27 | 355 ± 31 |
| Résultats exprimes en mg/dL ; valeurs moyennes ± SEM. | | | | |

Ces résultats montrent qu'après 6 semaines de traitement, aucun effet n'était observé avec l'acide (1S,3R,4R,5R)-3-{[(2E)-3-(3,4-dihydroxyphényl)-2-propenoyl]oxy}-1,4,5-trihydroxycyclohexanecarboxylique seul (groupe V63X35) et le (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle seul (groupe V63X54). Seule la combinaison des deux molécules (V63000) induisait une diminution de la glycémie à jeun (-26.2%, ANOVA $p < 0.05$, Tests de comparaisons multiples de Tukey: V63000 *versus* CONTRÔLE, $p < 0,05$). La metformine n'a eu aucun effet sur ce paramètre (CONTRÔLE, 481 mg/dL *versus* MET, 454 mg/dL, $p = 0.56$).

L'effet synergique sur la glycémie à jeun a été évalué selon la méthode de Colby SR décrite dans « Calculation of the synergistic and antagonitic responses of herbicide combinations » Weeds, 1967, 15:20-22. Un facteur > 1 indique l'existence d'un effet synergique. Un facteur < 1 indique l'existence d'un antagoniste.

Les calculs effectués étaient :

$$\text{Taux d'efficacité attendu} = V63X35 + V63X54 - (V63X35 * V63X54 / 100)$$

$$\text{Facteur de synergie (FS)} = (1 * \text{taux d'efficacité observé (\%, V63000)}) / \text{taux d'efficacité attendu (\%)}$$

V63X35 représente le pourcentage de variation de la glycémie pour le groupe traité par V63X35par rapport au groupe CONTRÔLE.

V63X54 représente le pourcentage de variation de la glycémie pour le groupe traité par V63X54 par rapport au groupe CONTRÔLE.

Le taux d'efficacité observé représente le % de variation de la glycémie à jeun du groupe V63000 par rapport au groupe CONTRÔLE.

Le résultat, illustré à la Figure 4, montre un effet synergique de la combinaison avec un FS = 3.58.

En conclusion, seule la combinaison V63000 a eu un effet sur la glycémie à jeun. Il s'agit d'un effet synergique, V63X35 et V63X54 n'ayant aucun effet sur ce paramètre. Par ailleurs, l'effet de la composition selon l'invention est supérieur à celui de la metformine, et cela malgré une dose moins importante (MET, 0.2% *versus* V63000 0.14% de l'alimentation). L'absence d'effet de la metformine sur ce modèle a déjà été reporté comme en témoigne notamment les travaux de Ohno T et al. (metformine 300 mg/kg/jour, PLoSONE 2015;10(4):e0124081). En effet, la metformine semble moins efficace, voire inefficace, chez les animaux présentant une sécrétion d'insuline inadéquate (GLUCOPHAGE®, metformine, monographie de produit 2009, Sanofi-Aventis, Canada). En d'autres termes, la metformine deviendrait ineffective lors d'un état avancé du diabète de type 2, caractérisé entre autres par une altération de la fonction insulino-sécrétrice du pancréas.

Les résultats sur l'insulinémie à jeun sont présentés dans le Tableau 3 et sur la Figure 5 qui illustrent la valeur médiane d'insulinémie à jeun après 6 semaines de traitement dans les différents groupes.

***Tableau 3. Valeurs médiane d'insulinémie après 6 semaines de traitement***

| CONTRÔLE | MET | V63X35 | V63X54 | V63000 |
|---|---|---|---|---|
| 23,03 | 21,89 | 22,39 | 25,44 | 49,97 |
| Résultats exprimés en ng/mL. | | | | |

On constate que toutes les valeurs médianes sont identiques à l'exception de la valeur médiane du groupe V63000 supérieure de 216% à celle du groupe CONTRÔLE (CONTRÔLE, 23.03 ng/mL *versus* ACHOLE 49.97 ng/mL). Ces résultats démontrent également un effet synergique de la combinaison de molécules, aucune différence n'étant observée entre les valeurs médianes des groupes CONTRÔLE, V63X35 et V63X54.

Le maintien d'une sécrétion d'insuline adéquate en réponse à une augmentation chronique de la glycémie est un enjeu médical de premier ordre pour le traitement du diabète de type 2 et de ses complications. L'augmentation de la valeur médiane dans le groupe V63000 démontre un meilleur niveau d'insuline en réponse à l'augmentation de la glycémie. En se rapportant à la Figure 1, le principe actif selon l'invention décale la courbe d'évolution de l'insulinémie vers la droite, traduisant une réelle efficacité pour le traitement ou la prévention du diabète de type 2, en particulier lors d'un stade avancé. Ces résultats permettent d'envisager un recul de l'insulino-thérapie chez les patients diabétiques de type 2, voire sa non mise en place.

Enfin, les résultats obtenus sur les triglycérides sanguins sont présentés dans le Tableau 4 et sur la Figure 6.

***Tableau 4. Triglycérides sérigues après 6 semaines de traitement***

| CONTRÔLE | MET | V63000 |
|---|---|---|
| 205,4 ± 17,8 | 132,6 ± 7,4 | 122,8 ± 14,0 |
| Résultats exprimés en mg/dL ; valeurs moyennes ± SEM. | | |

Ces résultats montrent que la metformine et le principe actif selon l'invention diminuent les taux de triglycérides sanguins de respectivement 35,6% et 40,4%.

L'effet de la metformine sur les triglycérides circulants est classiquement décrit dans les différentes notices d'utilisation des médicaments intégrant cette molécule. Le principe actif selon l'invention présente également un effet favorable sur le métabolisme des lipides permettant ainsi d'avoir une action globale sur le risque cardio-vasculaire.

**Revendications**

**1.** Principe actif pharmaceutique constitué exclusivement par l'association :

- de l'acide (1S,3R,4R,5R)-3-{[(2E)-3-(3,4-dihydroxyphényl)-2-propenoyl]oxy}-1,4,5-trihydroxycyclohexanecarboxylique, et
- du (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle,

pour une utilisation comme médicament ou produit vétérinaire.

**2.** Principe actif pharmaceutique pour une utilisation selon la revendication 1, dans la prévention et/ou le traitement d'au moins une maladie choisie parmi le diabète de type 2, les maladies du foie gras non alcoolique, les pathologies cardiovasculaires, la dyslipidémie, l'obésité et le syndrome métabolique.

**3.** Principe actif pharmaceutique pour une utilisation selon la revendication 2, **caractérisée en ce que** les pathologies cardiovasculaires sont choisies parmi les cardiopathies coronariennes, les maladies cérébro-vasculaires, les artériopathies périphériques et les thromboses veineuses profondes.

**4.** Principe actif pharmaceutique pour une utilisation selon la revendication 2, **caractérisée en ce que** la maladie du foie gras non alcoolique est la stéatose hépatique non alcoolique.

**5.** Principe actif pharmaceutique pour une utilisation selon l'une des précédentes revendications, **caractérisé en ce**

**que** le ratio acide (1S,3R,4R,5R)-3-{[(2E)-3-(3,4-dihydroxyphényl)-2-propenoyl]oxy}-1,4,5-trihydroxycyclohexanecarboxylique sur (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle en poids est compris entre 1/40 et 1/1.

6. Principe actif pharmaceutique pour une utilisation selon l'une des précédentes revendications, **caractérisé en ce que** les molécules de l'acide (1S,3R,4R,5R)-3-{[(2E)-3-(3,4-dihydroxyphényl)-2-propenoyl]oxy}-1,4,5-trihydroxycyclohexanecarboxylique et de (2S,3E,4S)-4-{2-[2-(3,4-Dihydroxyphényl)éthoxy]-2-oxoéthyl}-3-éthylidène-2-(β-D-glucopyranosyloxy)-3,4-dihydro-2H-pyrane-5-carboxylate de méthyle sont des molécules naturelles ou de synthèse par un procédé chimique et/ou biotechnologique.

7. Principe actif pharmaceutique pour une utilisation selon l'une des précédentes revendications, **caractérisé en ce qu'**il se présente sous forme solide ou liquide.

8. Principe actif pharmaceutique pour une utilisation selon l'une des précédentes revendications par voie orale, entérale, parentérale ou sous-cutanée.

9. Principe actif pharmaceutique pour une utilisation selon l'une des précédentes revendications au sein d'une composition pharmaceutique.

10. Composition pharmaceutique comprenant un principe actif pharmaceutique selon l'une des précédentes revendications pour une utilisation selon l'une des précédentes revendications, **caractérisée en ce qu'**elle se présente sous forme de comprimés, capsules, gélules, poudre, sachets, ampoules, solution pour compte-goutte ou solution injectable.

11. Composition pharmaceutique pour une utilisation selon la revendication 10, **caractérisée en ce qu'**elle comprend entre 0,5 et 100% du principe actif pharmaceutique en poids de matière sèche.

**Patentansprüche**

1. Pharmazeutischer Wirkstoff, ausschließlich bestehend aus der Kombination:

   - von (1S,3R,4R,5R)-3-{[(2E)-3-(3,4-Dihydroxyphenyl)-2-propenoyl]oxy}-1,4,5-trihydroxycyclohexancarbonsäure und
   - (2S,3E,4S)-4-{2-[2-(3,4-Dihydroxyphenyl)ethoxy]-2-oxoethyl}-3-ethyliden-2-(β-D-glucopyranosyloxy)-3,4-dihydro-2H-pyran-5-methylcarboxylat zur Verwendung als Medikament oder Tierarzneimittel.

2. Pharmazeutischer Wirkstoff zur Verwendung nach Anspruch 1 bei der Vorbeugung und/oder Behandlung von mindestens einer Krankheit, ausgewählt aus Typ-2-Diabetes, nichtalkoholischen Fettlebererkrankungen, kardiovaskulären Erkrankungen, Dyslipidämie, Obesität und metabolisches Syndrom.

3. Pharmazeutischer Wirkstoff zur Verwendung nach Anspruch 2, **dadurch gekennzeichnet, dass** die kardiovaskulären Erkrankungen ausgewählt sind aus koronaren Herzerkrankungen, zerebrovaskulären Erkrankungen, peripheren arteriellen Erkrankungen und tiefen Venenthrombosen.

4. Pharmazeutischer Wirkstoff zur Verwendung nach Anspruch 2, **dadurch gekennzeichnet, dass** die nichtalkoholische Fettlebererkrankung die nichtalkoholische Steatohepatitis ist.

5. Pharmazeutischer Wirkstoff zur Verwendung nach einem der vorhergehenden Ansprüche, **dadurch gekennzeichnet, dass** das Verhältnis (1S,3R,4R,5R)-3-{[(2E)-3-(3,4-Dihydroxyphenyl)-2-propenoyl]oxy}-1,4,5-trihydroxycyclohexancarbonsäure zu (2S,3E,4S)-4-{2-[2-(3,4-Dihydroxyphenyl)ethoxy]-2-oxoethyl}-3-ethyliden-2-(β-D-glucopyranosyloxy)-3,4-dihydro-2H-pyran-5-methylcarboxylat zwischen 1/40 und 1/1 liegt.

6. Pharmazeutischer Wirkstoff zur Verwendung nach einem der vorhergehenden Ansprüche, **dadurch gekennzeichnet, dass** die Moleküle der (1S,3R,4R,5R)-3-{[(2E)-3-(3,4-Dihydroxyphenyl) -2-propenoyl]oxy}-1,4,5-trihydroxycyclohexancarbonsäure und (2S,3E,4S)-4-{2-[2-(3,4-Dihydroxyphenyl)ethoxy]-2-oxoethyl}-3-ethyliden-2-(β-D-glucopyranosyloxy)-3,4-dihydro-2H-pyran-5-methylcarboxylatsäure natürliche Moleküle oder durch ein chemisches und/oder biotechnologisches Verfahren synthetisierte Moleküle sind.

7. Pharmazeutischer Wirkstoff zur Verwendung nach einem der vorhergehenden Ansprüche, **dadurch gekennzeichnet, dass** er in fester oder flüssiger Form vorliegt.

8. Pharmazeutischer Wirkstoff zur Verwendung nach einem der vorhergehenden Ansprüche auf oralem, enteralem, parenteralem oder subkutanem Weg.

9. Pharmazeutischer Wirkstoff zur Verwendung nach einem der vorhergehenden Ansprüche in einer pharmazeutischen Zusammensetzung.

10. Pharmazeutische Zusammensetzung, umfassend einen pharmazeutischen Wirkstoff nach einem der vorhergehenden Ansprüche zur Verwendung nach einem der vorhergehenden Ansprüche, **dadurch gekennzeichnet, dass** sie in Form von Tabletten, Kapseln, Gelatinekapseln, Pulver, Beuteln, Ampullen, Tropflösung oder injizierbarer Lösung vorliegt.

11. Pharmazeutische Zusammensetzung zur Verwendung nach Anspruch 10, **dadurch gekennzeichnet, dass** sie zwischen 0,5 und 100 Gew.-% des pharmazeutischen Wirkstoffs, bezogen auf das Gewicht der Trockensubstanz, umfasst.

## Claims

1. Pharmaceutical active ingredient consisting exclusively of the combination of:

   - (1S,3R,4R,5R)-3-{[(2E)-3-(3,4-dihydroxyphenyl)-2-propenoyl]oxy}-1,4,5-trihydroxycyclohexanecarboxylic acid, and
   - methyl (2S,3E,4S)-4-{2-[2-(3,4-dihydroxyphenyl)ethoxy]-2-oxoethyl}-3-ethylidene-2-(β-D-glucopyranosyloxy)-3,4-dihydro-2H-pyran-5-carboxylale for use as a drug or veterinary product.

2. Pharmaceutical active ingredient for use according to claim 1, in the prevention and/or treatment of at least one disease selected from type 2 diabetes, non-alcoholic fatty liver diseases, cardiovascular pathologies, dyslipidemia, obesity and metabolic syndrome.

3. Pharmaceutical active ingredient for use according to claim 2, **characterized in that** the cardiovascular pathologies are selected from coronary heart diseases, cerebrovascular diseases, peripheral arterial diseases and deep vein thrombosis.

4. Pharmaceutical active ingredient for use according to claim 2, **characterized in that** the non-alcoholic fatty liver disease is non-alcoholic hepatic steatosis.

5. Pharmaceutical active ingredient for use according to any of the preceding claims, **characterized in that** the weight ratio of (1S,3R,4R,5R)-3-{[(2E)-3-(3,4-dihydroxyphenyl)-2-propenoyl]oxy}-1,4,5-trihydroxycyclohexanecarboxylic acid to methyl (2S,3E,4S)-4-{2-[2-(3,4-dihydroxyphenyl)ethoxy]-2-oxoethyl}-3-ethylidene-2-(β-D-glucopyranosyloxy)-3,4-dihydro-2H-pyran-5-carboxylate is between 1:40 and 1:1.

6. Pharmaceutical active ingredient for use according to any of the preceding claims, **characterized in that** the molecules of (1S,3R,4R,5R)-3-{[(2E)-3-(3,4-dihydroxyphenyl)-2-propenoyl]oxy}-1,4,5-trihydroxycyclohexanecarboxylic acid and of methyl (2S,3E,4S)-4-{2-[2-(3,4-dihydroxyphenyl)ethoxy]-2-oxoethyl}-3-ethylidene-2-(β-D-glucopyranosyloxy)-3,4-dihydro-2H-pyran-5-carboxylate are natural molecules or synthetic molecules produced by a chemical and/or biotechnological process.

7. Pharmaceutical active ingredient for use according to any of the preceding claims, **characterized in that** it is in solid or liquid form.

8. Pharmaceutical active ingredient for use according to any of the preceding claims orally, enterally, parenterally or subcutaneously.

9. Pharmaceutical active ingredient for use according to any of the preceding claims in a pharmaceutical composition.

**10.** Pharmaceutical composition comprising a pharmaceutical active ingredient according to any of the preceding claims for use according to any of the preceding claims, **characterized in that** it is in the form of tablets, capsules, gel capsules, powder, sachets, ampoules, solution for droppers or injectable solution.

**11.** Pharmaceutical composition for use according to claim 10, **characterized in that** it comprises between 0.5 and 100 wt.% of dry matter of the pharmaceutical active ingredient.

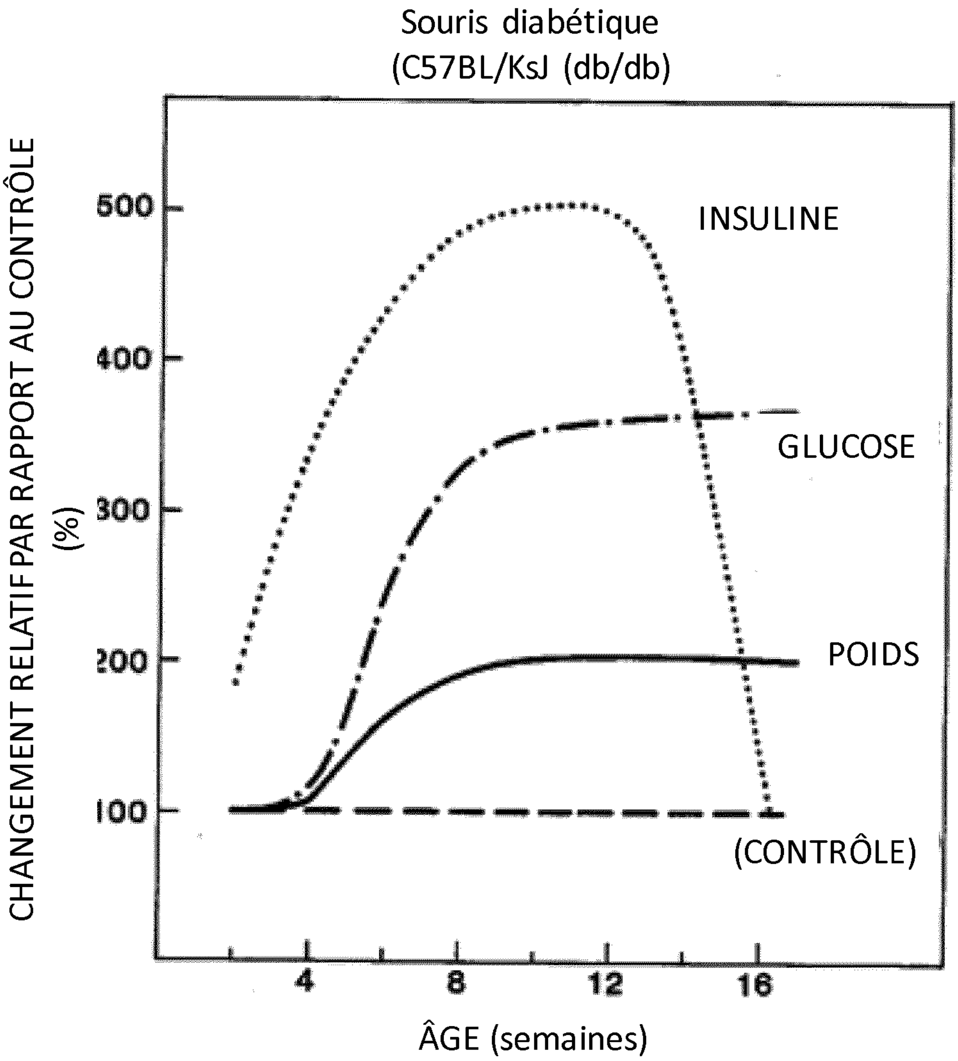
Souris diabétique
(C57BL/KsJ (db/db)
CHANGEMENT RELATIF PAR RAPPORT AU CONTRÔLE
(%)
500
400
300
200
100
INSULINE
GLUCOSE
POIDS
(CONTRÔLE)
4
8
12
16
ÂGE (semaines)

**Figure 1**

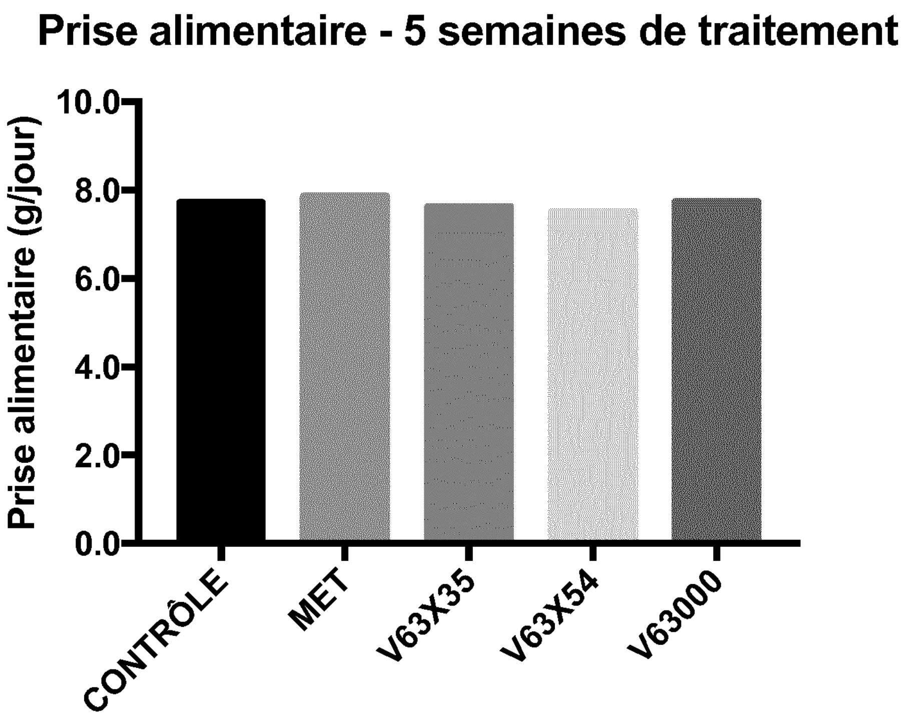
Prise alimentaire - 5 semaines de traitement
Prise alimentaire (g/jour)
10.0
8.0
6.0
4.0
2.0
0.0
CONTRÔLE
MET
V63X35
V63X54
V63000

Figure 2

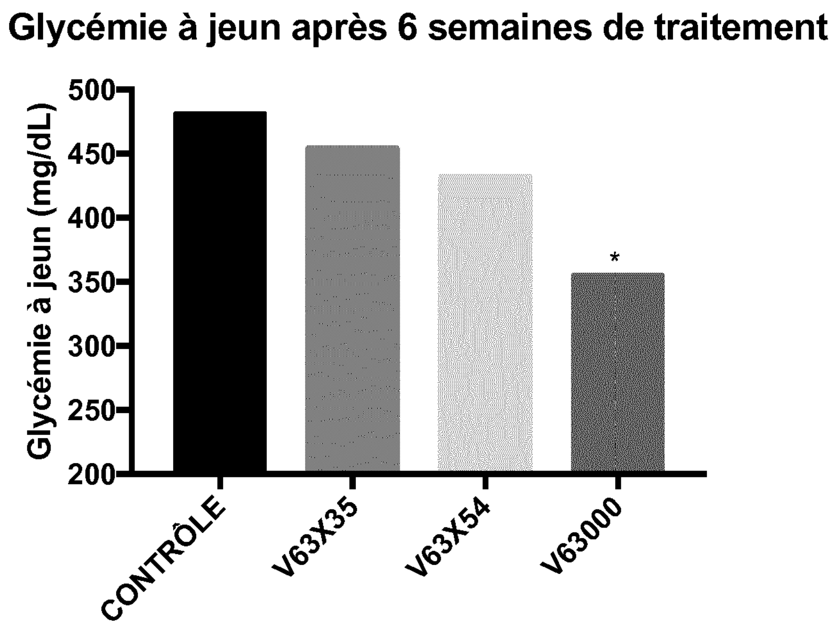


**ANOVA**
p < 0.05

**Tests de comparaisons multiples de Tukey**
V63000 *versus* CONTRÔLE, * p < 0.05

Figure 3

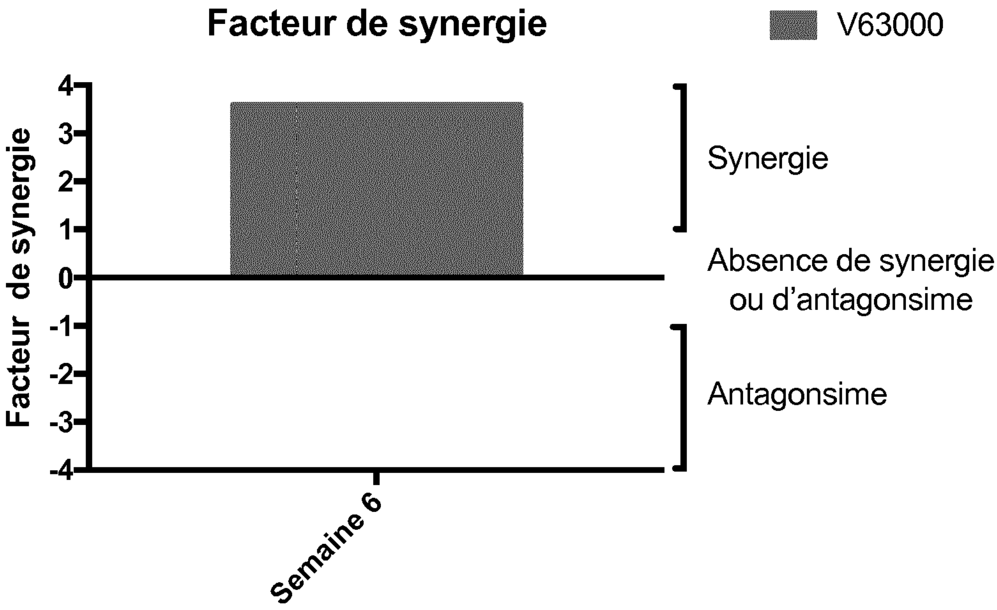


Figure 4

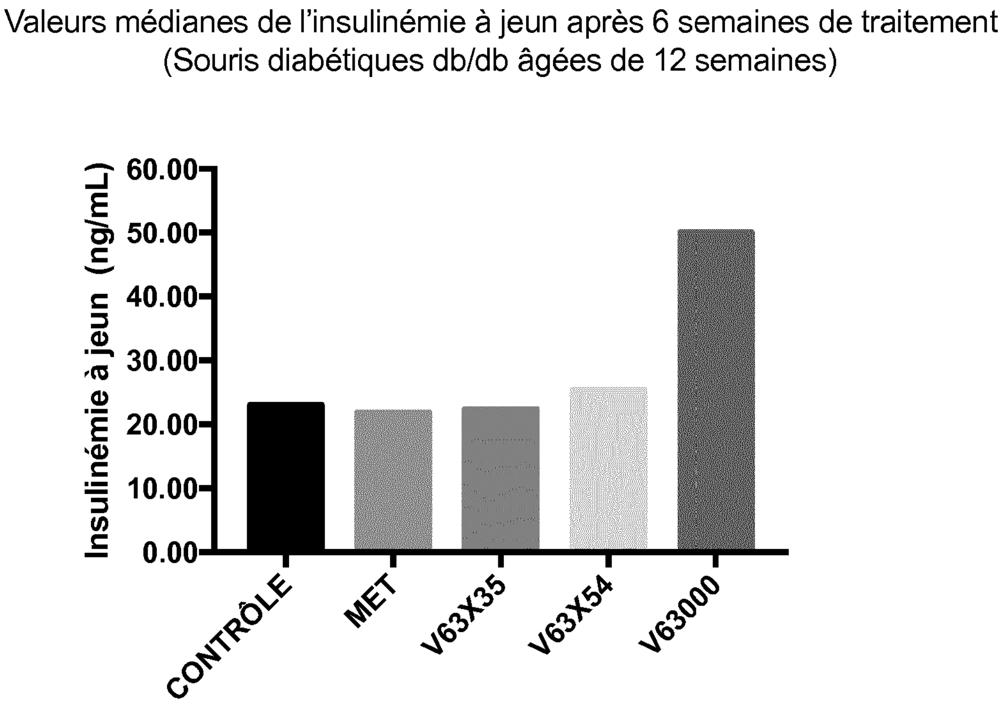


Figure 5

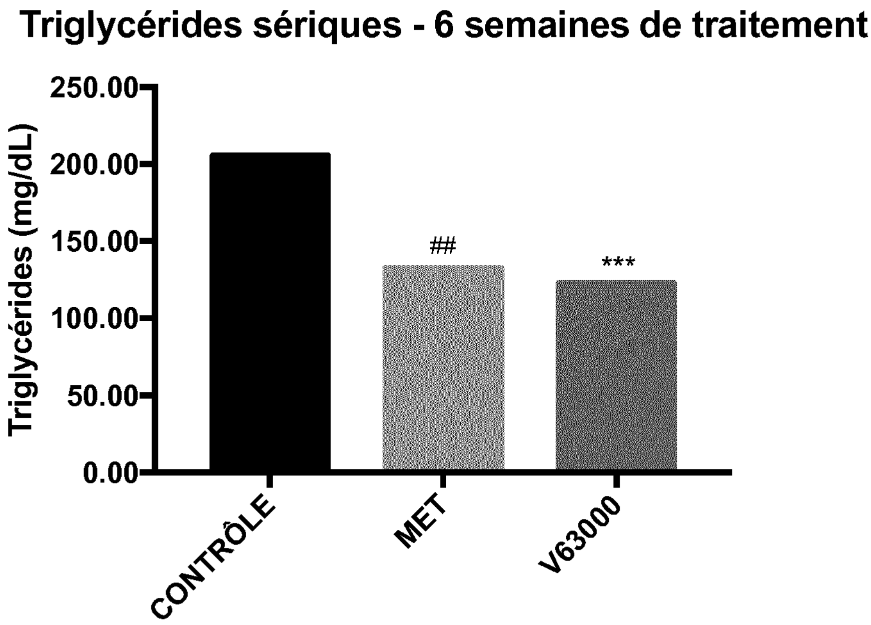


**ANOVA**
$p < 0.001$

**Tests de comparaisons multiples de Tukey**
MET *versus* CONTRÔLE, ## $p < 0.01$
V63000 *versus* CONTRÔLE, *** $p < 0.001$

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BOYLE JP et al.** *Popul Health Metr,* 2010, vol. 8, 29
- Atlas du diabète de la FID
- **BEIGI FI.** *N Eng J Med,* 2012, vol. 366, 1319-27
- *Diabetes Care,* 2015, vol. 38 (1), S33-S40
- **QASEEM A et al.** *Ann Intern Med,* 2012, vol. 156, 218-31
- **TAO H et al.** *Nat Med,* 2014, vol. 20 (11), 1263-69
- **NATHAN D et al.** *Diabetes Care,* 2009, vol. 32, 193-203
- **ANSTEE Q et al.** *Nat Rev Gastroenterol Hepatol,* 2013, vol. 10, 330-44
- **TARGHER G et al.** *Diabetes Care,* 2007, vol. 30, 1212-8
- **WILLIAMSON R et al.** *Diabetes Care,* 2011, vol. 34, 1139-44
- **ANGULO P N.** *Eng J Med,* 2002, vol. 346, 1221-31
- **NEUSCHWANDER-TETRI B et al.** *Hepatology,* 2003, vol. 37, 1202-19
- **ADAMS L et al.** *Gastroenterology,* 2005, vol. 129, 113-21
- **KOTRONEN A et al.** *J Clin Endocrinol Metab,* 2007, vol. 92, 3490-7
- **ADAMS L et al.** *Cmaj,* 2005, vol. 172, 899-905
- **KLEINER D et al.** *Hepatology,* 2005, vol. 41, 1313-21
- **BRUNT E.** *Nat Rev Gastroenterol Hepatol,* 2010, vol. 7, 195-203
- **VANNI E et al.** *Dig Liver Dis,* 2010, vol. 42, 320-30
- **DENKE M J.** *Manag Care Pharm,* 2003, vol. 9, 17-09
- **MARCHESINI G et al.** *Diabetes,* 2001, vol. 50, 1844-50
- **RATZIU V et al.** *J Hepatol,* 2010, vol. 53, 372-84
- **NEUSCHWANDER-TETRI BA et al.** *Hepatology,* 2003, vol. 37, 1202-19
- **ROESLER WJ et al.** *Mol Cell Biochem,* 1990, vol. 92 (2), 99-106
- **JOOST HG et al.** Animal Models in Diabetes Research. Humana Press
- **KOBAYASHI K et al.** *Metabolism: clinical and experimental,* 2000, vol. 49, 22-31
- **TAO H et al.** *Nat Med,* 2014, vol. 20 (11), 1263-1269
- **REAGAN-SHOW S et al.** *FASEB J,* 2008, vol. 22 (3), 659-61
- Calculation of the synergistic and antagonitic responses of herbicide combinations. *Weeds,* 1967, vol. 15, 20-22
- **OHNO T et al.** metformine 300 mg/kg/jour. *PLoS-ONE,* 2015, vol. 10 (4), e0124081